# EUROPEAN PATENT SPECIFICATION

(11) **EP 3 027 201 B1**
(45) Date of publication and mention of the grant of the patent: **13.06.2018**
(21) Application number: 14744867.4
(22) Date of filing: 31.07.2014
(51) Int. Cl.: A61K 38/17, A61K 35/545, A61K 35/34, A61P 9/10, C12N 5/077

(54) **GLYCOSYLATED PROTEIN OF AN EXTRA-CELLULAR MATRIX FOR USE IN A METHOD OF TREATING AN ISCHEMIC HEART OF A HUMAN OR ANIMAL SUBJECT IN NEED THEREOF**
GLYKOSYLIERTES PROTEIN EINER EXTRAZELLULAREN MATRIX ZUR VERWENDUNG BEI EINEM VERFAHREN ZUR BEHANDLUNG EINER HERZISCHÄMIE BEI EINEM MENSCHEN ODER EINEM TIER, DIE DIES BENÖTIGEN
PROTÉINE GLYCOSYLÉE D'UNE MATRICE EXTRACELLULAIRE DESTINÉE À ÊTRE UTILISÉE DANS UN PROCÉDÉ DE TRAITEMENT D'UN CÝUR ISCHÉMIQUE D'UN SUJET HUMAIN OU ANIMAL QUI EN A BESOIN

(30) Priority: 01.08.2013 EP 13178951; 06.05.2014 EP 14167172
(43) Date of publication of application: 08.06.2016
(73) Proprietor: Fraunhofer Gesellschaft zur Förderung der angewandten Forschung E.V., 80686 München (DE)
(72) Inventor: LAYLAND, Shannon, 72070 Tübingen (DE); SCHENKE-LAYLAND, Katja, 72070 Tübingen (DE); HOLEITER, Monika, 70569 Stuttgart (DE)
(74) Representative: Schrell, Andreas
(86) International application number: PCT/EP2014/066497
(87) International publication number: WO 2015/014938

(56) References cited:
- EP-A1- 2 594 635
- US-A1- 2007 014 773
- BAHARVAND H ET AL: "The effect of extracellular matrix on embryonic stem cell-derived cardiomyocytes", JOURNAL OF MOLECULAR AND CELLULAR CARDIOLOGY, ACADEMIC PRESS, GB, vol. 38, no. 3, 1 March 2005 (2005-03-01), pages 495-503, XP004762359, ISSN: 0022-2828, DOI: 10.1016/J.YJMCC.2004.12.011
- HOLEITER MONIKA ET AL: "Impact of human pluripotent stem cell-derived extracellular matrix proteins on cardiac cell fate decision", FASEB JOURNAL, vol. 27, April 2013 (2013-04), page 16.4, XP009172910, & JOINT ANNUAL MEETING OF THE ASPET/BPS AT EXPERIMENTAL BIOLOGY (EB); BOSTON, MA, USA; APRIL 20 -24, 2013
- ZHOU J ET AL: "Embryoid bodies formation and differentiation from mouse embryonic stem cells in collagen/Matrigel scaffolds", JOURNAL OF GENETICS AND GENOMICS, ELSEVIER BV, NL, vol. 37, no. 7, 1 July 2010 (2010-07-01), pages 451-460, XP027173437, ISSN: 1673-8527 [retrieved on 2010-07-01]

## Description

The present invention relates to a glycosylated protein of an extracellular matrix, preferably of a mammalian extracellular matrix for use in a method of treating an ischemic heart of a human or animal subject in need thereof, a pharmaceutical composition comprising said glycosylated protein and a method for the in vitro production of differentiated cardiomyocytes.

Ischemia is a restriction in blood supply which can result in damage or dysfunction of a tissue. Ischemia of a heart muscle due to a cardiovascular disease, in particular due to a myocardial infarction, is a major problem that has to be tackled by modern medicine. Heart failure is the number one cause of death in industrialized countries. Myocardial infarction typically results in fibrotic scar formation and permanently impaired cardiac function because, after a massive cell loss due to ischemia, the myocardial tissue lacks intrinsic regenerative capability. In subjects, which survived a myocardial infarction cardiac collagen accumulation takes place and stiffened collagen-rich scar tissue is formed, which could result in a hypertrophy of the remaining myocardium as well as pathological deformation of the left ventricle and finally in heart failure.

EP 1 730 265 A2 discloses a composition comprising a biodegradable gel-based matrix, at least one active agent and stem cells being able to differentiate into cardiac tissue.

EP 1 856 246 A2 discloses decellularized tissue-derived extracellular matrices and methods of generating and using the same. The methods of generating a decellularized matrix include the step of (a) subjecting the tissue to washes and a hypertonic buffer, (b) subjecting the tissue to an enzymatic proteolytic digestion with an enzyme, such as trypsin, and (c) removing all cellular components from the tissue using a detergent solution which includes Triton-X-100 and ammonium hydroxide.

EP 2 349 292 A2 discloses a composition comprising decellularized extracellular matrix derived from cardiac tissue.

Seif-Naraghi et al (Sci Transl Med, 2013, 5 (173), 173) disclose safety and efficacy of an injectable extracellular matrix hydrogel for treating myocardial infarction.

Sreejit et al. (Stem Cell Reviews and Reports, 2013, 9 (2), 158 to 171) disclose natural extracellular matrix as biomaterial for scaffold based cardiac regeneration using adult bone marrow derived stem cells.

Higuchi et al. (J Biosci Bioeng, 2013, 115(3), 320 to 5) disclose that heart extracellular matrix supports cardiomyocyte differentiation of mouse embryonic stem cells.

Hinderer et al. (Biomaterials, 2012, 33(21), 5259 to 66) disclose engineering of fibrillar decorin matrices for a tissue-engineered trachea.

Thus, various approaches have been provided to develop strategies for improving the extent and quality of regeneration of damage, in particular of ischemic heart tissue. However, none of the disclosed methods provide a satisfactory result. Accordingly, there is still an unmet demand for improved means and method to regenerate ischemic heart tissue.

The technical problem underlying the present invention is, in particular, to overcome the above identified disadvantages, preferably to provide a component, preferably a pharmaceutical composition, which improves the differentiation of stem cells, preferably pluripotent stem cells, cardiac stem cells and/or cardiac progenitor cells, into differentiated myocardiocytes, that means a higher number of cells should differentiate towards the cardiac lineage than without said component, preferably said pharmaceutical composition. In particular, by the use of the component and/or pharmaceutical composition to be provided the heart tissue of an animal or human subject that has suffered or is suffering ischemia should be superiorly regenerated or protected compared to a heart tissue of an animal or human subject not be treated with said component and/or pharmaceutical composition.

Said technical problem has been solved by the teaching of the independent claims.

In context with the present invention the term "cell" is understood in its broadest sense in the art and refers to a living body which is a structural unit of tissue of a multicellular organism, is surrounded by a membrane structure which isolates it from the outside, has the capability of self-replicating, and has genetic information and a mechanism for expressing it. Cells used herein may be naturally-occurring cells or artificially modified cells, for instance fusion cells and genetically modified cells.

In the context of the present invention, the term "stem cell" refers to a cell capable of giving rise to at least one type of a more specialized cell. A stem cell has the ability to self-renew, that is to go through numerous cycles of cell division while maintaining the undifferentiated state, and has the capacity to differentiate into specialized cell types. Usually, stem cells can regenerate an injured tissue.

Stem cells herein are preferably pluripotent cells or progenitor cells or mixtures thereof. The pluripotent cells are preferably induced pluripotent stem cells or embryonic stem cells or mixtures thereof. The progenitor cells are preferably multipotent cells. The multipotent cells are preferably cardiac progenitor cells, tissue stem cells, mesenchy-mal stem cells. Multipotent stem cells may originate from the heart, blood circulation or other tissues. Stem cells herein are preferably embryonic stem cells, induced pluripotent stem cells, or tissue stem cells, also called tissue-specific stem cell, adult stem cells or somatic stem cell.

"Pluripotent stem cells" in the context of the present invention are cells that can differentiate into cells derived from any of the three germ layers, for example, direct descendants of totipotent stem cells or induced pluripotent stem cells. Thus, the term "pluripotent stem cells" includes also induced pluripotent stem cells.

"Induced pluripotent stem cells" commonly abbreviated as iPS cells or iPSCs, refer to a type of pluripotent stem cell artificially prepared from a non-pluripotent cell, typically an adult somatic cell, or terminally differentiated cell, such as fibroblast, a hematopoietic cell, a myocyte, a neuron, an epidermal cell, or the like, by inserting certain genes, referred to as reprogramming factors or using other reprogramming methods.

"Embryonic stem cells" in the context of the present invention are cells derived from mammalian embryos or blastocysts, which are self-renewing and have the ability to yield many or all of the cell types present in a mature animal or human. Embryonic stem cells are pluripotent stem cells. Under defined conditions embryonic stem cells are capable of propagating themselves indefinitely. The pluripotency distinguishes embryonic stem cells from "adult stem cells" found in adults. While embryonic stem cells can generate all cell types in the body, adult stem cells are multipotent and can produce only a limited number of cell types.

Insofar as embryonic stem cells mentioned in the description of the invention are obtained directly or indirectly by the destruction of a human embryo, they do not form part of the claimed invention and are mentioned for reference purposes only.

"Adult stem cells", also known as somatic stem cells, are not pluripotent and non-terminally differentiated cells, found throughout the body after development, that multiply by cell division to replenish dying cells and regenerated damaged tissues. They can be found in juvenile as well as adult animals and human bodies. Adult stem cells are able to divide or self-renew indefinitely and generate all the cell types of an organ from which they originate, potentially regenerating the entire organ from a few cells. Adult stem cells possess the properties of self-renewal and multipotency. "Multipotency" means to have the ability to generate progenitors of several distinct cell types. Unlike embryonic stem cells, "cardiac stem cells" have a limited differentiation potential. Cardiac stem cells are tissue-specific stem progenitor cells, preferably tissue-specific progenitor cells within an adult mammalian heart.

"Progenitor cells" are cells with the capability of differentiating into several different cell types, but not any cell type. These cells are known to be multi-, bi- or unipotent. The most important difference between stem cells and progenitor cells is that stem cells can replicate indefinitely, whereas progenitor cells can divide only a limited number of times. Progenitor cells are in a later stage of cell differentiation in comparison to stem cells. Thus, in the process of differentiating cells progenitor cells are present between stem cells and fully differentiated cells.

In the context of the present invention "cardiomyocytes", also called cardiac muscle cells, are muscle cells that make up the cardiac muscle. Each cardiomyocyte contains myofibrils, which are long chains of sarcomeres, the contractile units of muscle cells. Cardiomyocytes show striations similar to those on skeletal muscle cells. Polyploidy and multinucleation are characteristic features of mammalian cardiomyocytes. The term "cardiomyocytes" includes cardiomyocyte-like cells that exhibit some but not all characteristics of mature cardiomyocytes, as well as mature and fully functional cardiomyocytes, preferably fully functional cardiomyocytes, which have all characteristics of cardiomyocytes as determined by morphology, characteristic protein marker expression, in vitro and in vivo functional assays.

The term "embryoid bodies" is understood as aggregates of differentiated and undifferentiated cells which can be preferably produced as described in Example 1, below, or by adding pieces of monolayered aggregated pluripotent stem cells to a substrate which they cannot adhere to.

As used herein, the term "carrier" refers to a diluent, adjuvant, excipient, or vehicle with which a compound is administered. Pharmaceutically acceptable carriers are preferably sterile liquids, such as water, oils and hydrogels. Water or aqueous solution saline solutions and aqueous dextrose and glycerol solutions are preferably employed as carriers, particularly for injectable solutions. Most preferably hydrogels are employed as carriers, particularly for injectable media.

In context with the present invention the term "in therapeutically effective amounts" means that at least 10%, preferably at least 20%, preferably at least 50%, preferably at least 60%, preferably at least 70%, preferably at least 80%, preferably at least 90%, preferably at least 99%, preferably 100% of the differentiable, preferably undifferentiated cells, preferably selected from the group consisting of stem cells, preferably pluripotent stem cells, cardiac stem cells, cardiac progenitor cells, multipotent stem cells and combinations thereof, are differentiated to cardiomyocytes. Preferably the at least one glycosylated protein is administrated to the human or animal subject in need thereof in therapeutically effective amounts, to achieve at least 1.1 times, preferably at least 1.2 times, preferably at least 1.3 times, preferably at least 1.4 times, preferably at least 1.5 times, preferably at least 2 times, preferably at least 5 times, preferably at least 10 times as many differentiable, preferably undifferentiated cells selected from the group consisting of stem cells, preferably embryonic stem cells, cardiac stem cells, cardiac progenitor cells, multipotent stem cells and combinations thereof, are differentiated to cardiomyocytes, compared to differentiable, preferably undifferentiated cells which are differentiated under the same conditions without the addition of the glycosylated protein, especially in a pharmaceutical composition.

A "biomolecule" which "encodes" a particular protein, is in the context of the present invention a nucleic acid molecule which is transcribed and optionally also translated into a gene product, for instance a polypeptide, in vitro or in vivo when placed under the control of appropriate regulatory sequences. The coding region may be present in a cDNA, genomic DNA, or RNA form. When present in a DNA form, the nucleic acid molecule may be single-stranded, that is the sense strand, or double-stranded. The boundaries of a coding region are determined by a start codon at the 5' (amino) terminus and a translation stop codon at the 3' (carboxy) terminus.

The term "recombinant DNA molecule" is understood in the context of the present invention as a DNA molecule that is comprised of segments of DNA joined together by means of molecular biological techniques.

The term "recombinantly produced protein" is understood in the context of the present invention as protein that is expressed from a recombinant DNA molecule. Recombinant DNA (rDNA) molecules are DNA sequences that are comprised of segments of DNA joined together by means of molecular biological techniques.

In context with the present invention the term "in vitro" is understood as an artificial environment and as processes or reactions that occur within an artificial environment. In vitro environments can preferably comprise test tubes and cell culture.

The term "extracellular matrix" means a protein-rich substance that is found in between cells in animal or human tissue and serves as a structural element in tissues. It typically comprises a complex mixture of polysaccharides and proteins secreted by cells. The extracellular matrix used can be isolated and treated in a variety of ways. Extracellular matrix material (ECM) is preferably derived from cells or stem cells, or isolated from small intestine submucosa, stomach submucosa, urinary bladder submucosa, tissue mucosa, dura mater, liver basement membrane, myocardium, pericardium or other tissues. Following isolation and treatment, it is commonly referred to as extracellular matrix or ECM material.

The term "pharmaceutical composition" is understood as formulation which provides some therapeutic and/or beneficial effect, preferably produces a localized or systemic effect or effects in animals or humans, preferably in warm blooded mammals, preferably in humans.

In context with the present invention the term "subject in need thereof" is understood as any animal or human subject that has suffered or is suffering from ischemia, preferably from one or more of the following: myocardial infarction, chronic heart failure, ischemic heart disease, coronary artery disease, diabetic heart disease, thrombotic stroke or embolic stroke. The animal or human subject is preferably a warm blooded mammal, preferably a human.

The term "administration" means that a pharmaceutical composition or an active agent formulation is provided to a treatment site, for instance a damaged tissue, through any method appropriate to deliver the functional agent or formulation or composition to the treatment site. Delivery methods are preferably direct injection, percutaneous delivery and topical application at the treatment site.

In the context of the present invention the terms "treatment" and "treating" mean obtaining a desired pharmaceutical and/or physiological effect. Preferably, the effect is therapeutic in terms of partially or completely curing ischemia. The term "treatment" as used herein covers any treatment of heart tissue suffering from ischemia and/or heart defects and/or heart dysfunction caused by ischemia in a human or animal, preferably a mammal, particularly a vertebrate and more preferably a human, and includes regenerating and/or repairing at least partially the heart or heart tissue dysfunction.

The term "ischemia", also called cardiac ischemia, is understood in the context with the present invention as a condition that may occur in the heart tissue that is suffering a lack of oxygen supply and/or supply with metabolites which occurs when there is an imbalance between oxygen supply and demand, due to inadequate perfusion, that leads to insufficient oxygen in the heart tissue. Also medical interventions such as the interruption of the blood flow, for instance during bypass surgery may lead to ischemia. Cardiac ischemia includes a broad variety of conditions, from silent ischemia to stable or unstable angina to myocardial infarction (AMI or "heart attack").

In the context of the present invention, an agent acts "cardioinductive" if by the addition of said agent a statistically significantly higher amount of undifferentiated cells are differentiated to cardiomyocytes, compared to the same differentiable, preferably undifferentiated, cells differentiated under the same conditions without the addition of said agent.

In the context of the present invention "statistically significant" means that a p-value of less than 0.05 is obtained by carrying out an analysis of variance (ANOVA) followed by the Fisher's least significant difference (LSD) test. In the figures, the statistical significance is denoted by one (*) or two (**) stars.

An agent acts preferably as cardioinductive agent if at least 10% more, preferably at least 20% more, preferably at least 30% more, preferably at least 40% more, preferably at least 50% more, preferably at least 60% more, preferably at least 70% more, preferably at least 80% more, preferably at least 90% more, preferably at least 100% more, preferably at least 150% more, preferably at least 200% more, preferably at least 300% more differentiable, preferably undifferentiated cells are differentiated to cardiomyocytes by the addition of said agent, compared to the same differentiable cells, preferably undifferentiated cells, differentiated under the same conditions, preferably according to Example 5, without the addition of said agent.

In the context of the present invention, an agent acts "cardioprotective" if by the addition of said agent a statistically significantly higher amount of cells, preferably cardiac cells, preferably cardiomyocytes, survive an oxygen depletion of at least 20 minutes to 60 minutes, preferably of 20 minutes to 60 minutes, preferably of 20 minutes.

In a preferred embodiment of the present invention, an agent is a cardioprotective agent if 50% or more cells, preferably cardiac cells, preferably cardiomyocytes, survive in vitro an oxygen depletion of 20 minutes, by adding said agent directly after the completion of the oxygen depletion and subsequently subjecting said cells to an incubator filled with air for two days at 37° C compared to the same cells, preferably cardiac cells, preferably cardiomyocytes, subjected to the same conditions without the addition of said agent.

In a further preferred embodiment of the present invention, an agent is a cardioprotective agent if 40% or more cells, preferably cardiac cells, preferably cardiomyocytes, survive in vitro an oxygen depletion of 30 minutes, by adding said agent directly after the completion of the oxygen depletion and subsequently subjecting said cells to an incubator filled with air for two days at 37° C compared to the same cells, preferably cardiac cells, preferably cardiomyocytes, subjected to the same conditions without the addition of said agent.

In a further preferred embodiment of the present invention, an agent is a cardioprotective agent if 30% or more cells, preferably cardiac cells, preferably cardiomyocytes, survive in vitro an oxygen depletion of 45 minutes, by adding said agent directly after the completion of the oxygen depletion and subsequently subjecting said cells to an incubator filled with air for two days at 37° C compared to the same cells, preferably cardiac cells, preferably cardiomyocytes, subjected to the same conditions without the addition of said agent.

In a further preferred embodiment of the present invention, an agent is a cardioprotective agent if 20% or more cells, preferably cardiac cells, preferably cardiomyocytes, survive in vitro an oxygen depletion of 60 minutes, by adding said agent directly after the completion of the oxygen depletion and subsequently subjecting said cells to an incubator filled with air for two days at 37° C compared to the same cells, preferably cardiac cells, preferably cardiomyocytes, subjected to the same conditions without the addition of said agent.

The term "ejection fraction" is understood as the fraction of the end-diastolic volume ejected with each heartbeat, that is stroke volume divided by end-diastolic volume. The end-diastolic volume is the volume of blood within a ventricle immediately before a contraction of the heart muscle. The stroke volume is the difference between the end-diastolic volume and end-systolic volume. The end-systolic volume is the volume of blood left in a ventricle at the end of the contraction of a heart muscle.

In accordance with the present invention nidogen-1 is the glycosylated protein of the extracellular matrix, preferably of the mammalian extracellular matrix used in the method of treating the ischemic heart. "Nidogen-1", also called entactin, is a sulfated multidomain glycoprotein component of basement membranes, alongside other components such as collagen type IV, proteoglycans, such as heparan sulfate and glycosaminoglycans, laminin and fibronectin. Nidogen-1 binds calcium ions and this calcium-binding activity of nidogen-1 may play a role in the matrix assembly process. Its major function appears to be the assembly of the basement membrane. Nidogen-1 is preferably a mammalian nidogen-1, preferably a human or humanized nidogen-1.

The technical problem of the present invention is particularly solved by a glycosylated protein of an extracellular matrix, preferably of a mammalian extracellular matrix for the use in a method of treating an ischemic heart of a human or animal subject in need thereof, wherein the glycosylated protein of the extracellular matrix, preferably the mammalian extracellular matrix is nidogen-1.

It has been surprisingly found that the extracellular matrix protein nidogen-1 plays a significant role in the cardiovascular differentiation of differentiable, preferably undifferentiated cells selected from the group consisting of stem cells, preferably embryonic stem cells, cardiac stem cells, cardiac progenitor cells, multipotent stem cells and combinations thereof. Said protein can be used for achieving the differentiation of stem cells, preferably pluripotent stem cells and/or progenitor cells into the cardiovascular lineage. Accordingly, said protein is cardioinductive.

Thus, by using the extracellular matrix protein nidogen-1, conditions and an environment for the differentiation of the stem cells and/or progenitor cells are provided in the ischemic heart, which leads to the generation of cardiomyocytes.

Nidogen-1 is used as cardioinductive agent, preferably as agent differentiating differentiable, preferably undifferentiated cells selected from the group consisting of stem cells, preferably embryonic stem cells, cardiac stem cells, cardiac progenitor cells, multipotent stem cells and combinations thereof, to cardiomyocytes.

By the use of nidogen-1 the percentage of CTNT (cardiac troponin T)-positive cells is significantly increased in comparison with a control sample of cells which have been differentiated without using nidogen-1, preferably after a period of time of at least 5 days, preferably at least or exactly 10 days of cardiovascular differentiation, when differentiable, preferably undifferentiated cells selected from the group consisting of stem cells, preferably embryonic stem cells, cardiac stem cells, cardiac progenitor cells, multipotent stem cells and combination thereof, are differentiated. This effect is at least present in-vitro. The injection of nidogen-1 leads furthermore in an in-vivo mouse model to an improvement of the ejection fraction compared to a control mouse model which has not been treated with nidogen-1 at the same time after heart attack, preferably after at least two or four weeks after the heart attack.

Thus, by the addition of nidogen-1 the ejection fraction substantially improves, that is increases, or remains equal, preferably over a period of at least two days, preferably at least two weeks, preferably at least four weeks compared to an ejection fraction of a heart which has not been treated with nidogen-1 directly after suffering a heart attack. Accordingly, nidogen-1 acts preferably as cardioprotective agent.

In the context with the present invention, nidogen-1 acts preferably as cardioinductive and/or cardioprotective agent, preferably depending on the cells nidogen-1 is brought in contact with. If differentiable, preferably undifferentiated cells, are brought in contact with nidogen-1, nidogen-1 acts preferably as cardioinductive agent. If differentiated cells, preferably cardiac cells, preferably cardiomyocytes, are brought in contact with nidogen-1, nidogen-1 acts preferably as cardioprotective agent. If both cell types, namely differentiable and differentiated cells, are brought in contact with nidogen-1, nidogen-1 acts preferably as cardioinductive and cardioprotective agent.

Cardiac troponin T is a specific marker for cardiomyocytes. Accordingly, by the use of nidogen-1 the percentage of Cardiac troponin T and cardiomyocytes is preferably also significantly increased in comparison with a control sample of cells which have been differentiated without using nidogen-1.

In a preferred embodiment of the present invention the glycosylated protein of the extracellular matrix, preferably of the mammalian extracellular matrix is a recombinantly produced protein, preferably a recombinantly produced human protein. By recombinant production the glycosylated protein of the extracellular matrix, preferably of the mammalian extracellular matrix can be produced in a reproducible and controlled way and can be administrated to the subject in need thereof in a defined concentration.

The recombinant production of the human protein nidogen-1 takes preferably place in host cell lines, preferably Chinese hamster ovary host cell lines (CHO-host cell lines). CHO-host cell lines have been approved by the Food and Drug Administration (FDA) for the recombinant production of protein therapeutics due to their high safety level. For clinical studies the production of the recombinantly produced, preferably human, protein can further be adjusted to good manufacturing practice (GMP) conditions.

The glycosylated protein and/or the pharmaceutical composition according to the present invention are preferably contamination-free, preferably free of viruses, prions and other not yet known risks, which can be transferred to the subject in need thereof, in particular free of viruses and prions.

Preferably, nidogen-1 is the sole protein used in a method according to the present invention. In this preferred embodiment, nidogen-1 is also the sole protein present in the pharmaceutical composition.

Preferably, nidogen-1 is the sole glycosylated protein used in a method according to the present invention. In this preferred embodiment, nidogen-1 is also the sole glycosylated protein present in the pharmaceutical composition.

Preferably, nidogen-1 is the sole extracellular matrix component used in a method according to the present invention. In this preferred embodiment, nidogen-1 is also the sole extracellular matrix component present in the pharmaceutical composition.

Preferably, nidogen-1 is the sole component of the basement membrane used in a method according to the present invention. In this preferred embodiment, nidogen-1 is also the sole component of the basement membrane present in the pharmaceutical composition.

Preferably, nidogen-1 is the sole structural protein of the extracellular matrix scaffold used in a method according to the present invention. In this preferred embodiment, nidogen-1 is also the sole structural protein of the extracellular matrix scaffold present in the pharmaceutical composition. The structural proteins of the extracellular matrix scaffold are preferably fibronectin, elastin, collagen and fibrillins.

Preferably, nidogen-1 is the sole functional protein of the extracellular matrix scaffold used in a method according to the present invention. In this preferred embodiment, nidogen-1 is also the sole functional protein of the extracellular matrix scaffold present in the pharmaceutical composition.

The use of nidogen-1 in a method according to the present invention, preferably as cardioinductive and/or cardioprotective agent, is preferably free of decorin. In this preferred embodiment, the pharmaceutical composition is also free of decorin.

The use of nidogen-1 in a method according to the present disclosure, preferably as cardioinductive and/or cardioprotective agent, is preferably free of collagen. In this preferred embodiment, the pharmaceutical composition is also free of collagen.

The use of nidogen-1 in a method according to the present disclosure preferably as cardioinductive and/or cardioprotective agent, is preferably free of decorin and collagen. In this preferred embodiment, the pharmaceutical composition is also free of decorin and collagen.

The use of nidogen-1 in a method according to the present disclosure, preferably as cardioinductive and/or cardioprotective agent, is preferably free of laminin, collagen IV and heparan sulfate. In this preferred embodiment, the pharmaceutical composition is also free of laminin, collagen IV and heparan sulfate.

The use of nidogen-1 in a method according to the present disclosure, preferably as cardioinductive and/or cardioprotective agent, is preferably free of laminin, collagen IV and heparan sulfate. In this preferred embodiment, the pharmaceutical composition is also free of laminin, collagen IV and heparan sulfate.

The use of nidogen-1 in a method according to the present disclosure preferably as cardioinductive and/or cardioprotective agent, is preferably free of collagen I and III, elastin, laminin, cytoplasmic domain-44 (CD44), hyaluronan, syndecan, basic fibroblast growth factor (bFGF), hepatocyte growth factor (HGF), platelet-derived growth factor (PDGF), vascular endothelial growth factor (VEGF), fibronectin (Fn), tenascin, heparin, heparan sulfate, chondroitin sulfate B, integrins, decorin, and transforming growth factor beta (TGF beta). In this preferred embodiment, the pharmaceutical composition is also free of collagen I and III, elastin, laminin, cytoplasmic domain-44 (CD44), hyaluronan, syndecan, basic fibroblast growth factor (bFGF), hepatocyte growth factor (HGF), PDGF, platelet-derived growth factor (PDGF), vascular endothelial growth factor (VEGF) fibronectin (Fn), tenascin, heparin, heparan sulfate, chondroitin sulfate B, integrins, decorin, and transforming growth factor beta (TGF beta).

The use of nidogen-1 in a method according to the present disclosure preferably as cardioinductive and/or cardioprotective agent, is preferably free of at least one component selected from the group consisting of a collagen, a proteoglycan, a glycosaminoglycan (GAG) chain, a further glycoprotein, a growth factor, a cytokine, a cell-surface associated protein, a cell adhesion molecule (CAM), an angiogenic growth factor, an endothelial ligand, a matrikine, a matrix metalloprotease, a cadherin, an immunoglobin, a fibril collagen, a non-fibrillar collagen, a basement membrane collagen, a multiplexin, a small-leucine rich proteoglycan, decorin, biglycan, a fibromodulin, keratocan, lumican, epiphycan, a heparan sulfate proteoglycan, perlecan, agrin, testican, syndecan, glypican, serglycin, selectin, a lectican, aggrecan, versican, nuerocan, brevican, cytoplasmic domain-44 (CD-44), macrophage stimulating factor, amyloid precursor protein, heparin, chondroitin sulfate C, chondroitin sulfate B (dermatan sulfate), chondroitin sulfate A, heparan sulfate, hyaluronic acid, fibronectin (Fn), tenascin, elastin, fibrillin, laminin, fibulin I, fibulin II, integrin, a transmembrane molecule, platelet derived growth factor (PDGF), epidermal growth factor (EGF), transforming growth factor alpha (TGF-alpha), transforming growth factor beta (TGF-beta), fibroblast growth factor-2 (FGF-2) (also called basic fibroblast growth factor (bFGF)), thrombospondin, osteopontin, angiotensin converting enzyme (ACE), and vascular epithelial growth factor (VEGF).

In this preferred embodiment, the pharmaceutical composition is also free of at least one component selected from the group consisting of a collagen, a proteoglycan, a glycosaminoglycan (GAG) chain, a further glycoprotein, a growth factor, a cytokine, a cell-surface associated protein, a cell adhesion molecule (CAM), an angiogenic growth factor, an endothelial ligand, a matrikine, a matrix metalloprotease, a cadherin, an immunoglobin, a fibril collagen, a non-fibrillar collagen, a basement membrane collagen, a multiplexin, a small-leucine rich proteoglycan, decorin, biglycan, a fibromodulin, keratocan, lumican, epiphycan, a heparan sulfate proteoglycan, perlecan, agrin, testican, syndecan, glypican, serglycin, selectin, a lectican, aggrecan, versican, nuerocan, brevican, cytoplasmic domain-44 (CD-44), macrophage stimulating factor, amyloid precursor protein, heparin, chondroitin sulfate C, chondroitin sulfate B (dermatan sulfate), chondroitin sulfate A, heparan sulfate, hyaluronic acid, fibronectin (Fn), tenascin, elastin, fibrillin, laminin, fibulin I, fibulin II, integrin, a transmembrane molecule, platelet derived growth factor (PDGF), epidermal growth factor (EGF), transforming growth factor alpha (TGF-alpha), transforming growth factor beta (TGF-beta), fibroblast growth factor-2 (FGF-2) (also called basic fibroblast growth factor (bFGF)), thrombospondin, osteopontin, angiotensin converting enzyme (ACE), and vascular epithelial growth factor (VEGF).

Especially if a recombinantly produced human protein is used, it will not be recognised as being foreign by the human immune system, which would result in an adverse immune reaction. The same applies to a recombinantly produced protein of a specific animal, when administered to the same specific animal.

In a preferred embodiment of the present invention, the pharmaceutical composition and/or the glycosylated protein will not only be administrated, preferably injected, into the damaged heart tissue, but also to the adjacent regions thereof.

Preferably, the pharmaceutical composition or the glycosylated protein nidogen-1 will be administrated, preferably solely, into the heart tissue suffered ischemia. Preferably, the nidogen-1 or the pharmaceutical composition will be administrated, preferably solely, into the adjacent regions of the heart tissue suffered ischemia. Preferably, the nidogen-1 or the pharmaceutical composition will be administrated, preferably solely, into the infarct border region of the tissue suffered ischemia. Preferably, the pharmaceutical composition or the glycosylated protein nidogen-1 will be administrated, preferably solely, into the heart tissue suffered ischemia or into the infarct border region of the tissue suffered ischemia. The infarct border region is preferably defined as a region surrounding the heart tissue suffered ischemia. The infarct border region has preferably a width of at most 1 cm, preferably at most 0.5 cm, preferably at most 1 mm. The infarct border region has preferably a width of at least 1 µm, preferably at least 50 µm, preferably at least 100 µm. The infarct border region has preferably a width of from 1 µm to 1 cm, preferably of from 50 µm to 0.5 cm.

The perpendicular distance of the local position of the nidogen-1 administration into the infarct border issue from a tangent plane of a heart infarct tissue interface separating a tissue suffered ischemia from intact tissue, also called surface of the heart tissue suffered ischemia, is preferably at most 1 mm, preferably at most 0.5 mm, preferably at most 100 µm, preferably at most 50 µm, preferably at most 10 µm, preferably at most 1 µm.

The technical problem of the present invention is also particularly solved by a pharmaceutical composition for use in a method of treating an ischemic heart of a human or animal subject in need thereof, comprising a glycosylated protein of an extracellular matrix, preferably a mammalian extracellular matrix, which is nidogen-1.

In a preferred embodiment of the present invention the glycosylated protein of an extracellular matrix, preferably of a mammalian extracellular matrix, which is nidogen-1, is used and/or administered in therapeutically effective amounts. The present invention relates in a preferred embodiment to a pharmaceutical composition according to the present disclosure comprising further at least one pharmaceutically acceptable carrier.

The present invention relates in a preferred embodiment to a glycosylated protein according to the present disclosure or pharmaceutical composition according to the present disclosure, wherein the treating of the ischemic heart comprises regenerating and/or protecting the heart tissue.

The present invention relates in a preferred embodiment to a pharmaceutical composition according to the present disclosure, wherein the protein of the extracellular matrix, preferably of the mammalian extracellular matrix, namely nidogen-1, is comprised in a two or three dimensional scaffold. The three dimensional scaffold is preferably a mesh, a hydrogel, an electro-spun, bioprinted or biosprayed three-dimensional scaffold or a native tissue scaffold.

The present invention relates in a preferred embodiment to a pharmaceutical composition according to the present disclosure wherein the protein of the extracellular matrix, preferably of the mammalian extracellular matrix, namely nidogen-1, is comprised in a hydrogel.

The present invention relates in a preferred embodiment to a pharmaceutical composition according to the present disclosure, wherein the protein of the extracellular matrix, preferably of the mammalian extracellular matrix, namely nidogen-1, is comprised in an electro-spun three-dimensional scaffold.

The present invention relates in a preferred embodiment to a pharmaceutical composition according to the present disclosure, wherein the protein of the extracellular matrix, preferably of the mammalian extracellular matrix, namely nidogen-1, is present together with differentiable, preferably undifferentiated cells selected from the group consisting of stem cells, preferably embryonic stem cells, cardiac stem cells, cardiac progenitor cells, multipotent stem cells and combinations thereof.

The technical problem of the present invention is particularly solved by the use of a glycosylated protein of an extracellular matrix, preferably of a mammalian extracellular matrix, which is nidogen-1 for the treatment of an ischemic heart of a human or animal subject in need thereof.

Thus, the present invention also relates to the use of the above-identified glycosylated protein of an extracellular matrix, preferably of a mammalian extracellular matrix for the preparation of a pharmaceutical composition for the treatment of ischemic heart of a human or animal subject in need thereof.

The technical problem of the present invention is also particularly solved by the use of a pharmaceutical composition for treating an ischemic heart of a human or animal subject in need thereof, comprising a glycosylated protein of an extracellular matrix, preferably of a mammalian extracellular matrix, which is nidogen-1.

The present invention relates in a preferred embodiment to a use according to the present invention, wherein the treating of the ischemic heart comprises regenerating the heart tissue.

The present invention relates in a preferred embodiment to a use according to the present invention, wherein the protein of the extracellular matrix, preferably of the mammalian extracellular matrix, namely nidogen-1 is administrated to the subject in need thereof in a two or three dimensional scaffold. The three dimensional scaffold is preferably a mesh, a hydrogel, an electro-spun, bioprinted or biosprayed three-dimensional scaffold or a native tissue scaffold, preferably a hydrogel and/or an electro-spun three-dimensional scaffold.

The present invention relates in a preferred embodiment to a use according to the present invention, wherein the one protein of the extracellular matrix, preferably of the mammalian extracellular matrix, namely nidogen-1 is administrated to the subject in need thereof together with differentiable, preferably undifferentiated cells selected from the group consisting of stem cells, preferably embryonic stem cells, cardiac stem cells, cardiac progenitor cells, multipotent stem cells and combinations thereof.

In the preferred embodiment the one protein of the extracellular matrix, preferably of the mammalian extracellular matrix, namely nidogen-1 is provided in injectable form for injection in the ischemic heart of the subject in need thereof.

The present invention relates in a preferred embodiment to a use according to the present invention, wherein the one protein of the extracellular matrix, preferably of the mammalian extracellular matrix, namely nidogen-1 is administrated by injection in the ischemic heart of the subject in need thereof.

The present invention relates in a preferred embodiment to a use according to the present invention, wherein the protein of the extracellular matrix, preferably of the mammalian extracellular matrix, namely nidogen-1 is a recombinantly produced protein.

The technical problem of the present invention is also particularly solved by a method for the in vitro production of differentiated cardiomyocytes, wherein differentiable, preferably undifferentiated cells selected from the group consisting of stem cells, preferably embryonic stem cells, cardiac stem cells, cardiac progenitor cells, multipotent stem cells and combinations thereof are cultured with at least one biomolecule being or encoding a glycosylated protein of an extracellular matrix, preferably of a mammalian extracellular matrix , which is nidogen-1, so as to obtain differentiated cardiomyocytes. The biomolecule encoding the glycosylated protein of the extracellular matrix, preferably of the mammalian extracellular matrix is preferably present in host cells translating said biomolecule into the glycosylated protein nidogen-1.

Preferably, the differentiable cells are cultivated on a plate coated with nidogen-1. Preferably, the plate is coated with a 0.05 to 5 % by weight, preferably 0.09 to 2 % by weight, preferably 0.1 % by weight gelatine solution comprising preferably 1 to 500 µg/ml, preferably 20 to 200 µg/ml, preferably 50 µg/ml nidogen-1. As solvent for the gelatine solution water and/or phosphate buffered saline is, preferably solely, used.

In a preferred embodiment of the present invention, the cardiac stem cells are cardiac adult stem cells.

The technical problem of the present invention is also particularly solved by a method for treatment of an ischemic heart of a human or animal subject in need thereof by administering a glycosylated protein of an extracellular matrix, preferably of a mammalian extracellular matrix, which is nidogen-1.

The technical problem of the present invention is also particularly solved by a method for treatment of an ischemic heart of a human or animal subject in need thereof by administering a pharmaceutical composition comprising at least one glycosylated protein of an extracellular matrix, preferably of a mammalian extracellular matrix, which is nidogen-1.

Preferably, the treatment of the ischemic heart comprises regenerating and/or protecting the heart tissue.

The, preferably recombinantly produced, protein of the extracellular matrix, preferably of the mammalian extracellular matrix, namely nidogen-1, is preferably administrated to the subject in need thereof together with or in a two or three dimensional scaffold, preferably by injection into the ischemic heart of the subject in need thereof. The three dimensional scaffold is preferably a mesh, a hydrogel, an electro-spun, bioprinted or biosprayed three-dimensional scaffold or a native tissue scaffold, preferably a hydrogel or an electro-spun three-dimensional scaffold.

More preferably the at least one protein of the extracellular matrix, preferably of the mammalian extracellular matrix, namely nidogen-1, is administrated to the subject in need thereof together with differentiable, preferably undifferentiated cells selected from the group consisting of stem cells, preferably embryonic stem cells, cardiac stem cells, cardiac progenitor cells, multipotent stem cells and combinations thereof.

Most preferably the at least one protein of the extracellular matrix, preferably of the mammalian extracellular matrix, namely nidogen-1, is comprised together with differentiable, preferably undifferentiated cells, selected from the group consisting of stem cells, preferably embryonic stem cells, cardiac stem cells, cardiac progenitor cells, multipotent stem cells and combinations thereof, in a two or three dimensional scaffold, preferably a mesh, a hydrogel, an electro-spun, bioprinted or biosprayed three-dimensional scaffold or a native tissue scaffold, preferably a hydrogel or an electro-spun three-dimensional scaffold.

The technical problem of the present invention is also particularly solved by a kit comprising (a) a glycosylated protein of an extracellular matrix, preferably of a mammalian extracellular matrix, which is nidogen-1, and (b) cells selected from the group consisting of stem cells, preferably embryonic stem cells, cardiac stem cells, cardiac progenitor cells, multipotent stem cells and combinations thereof. Preferably, the kit further comprises an extracellular matrix, preferably a mammalian extracellular matrix. Preferably, the kit comprising (a) and (b) further comprises a two or three dimensional scaffold.

In the method of treating an ischemic heart of an animal or human subject in need thereof according to the present disclosure, wherein the heart that has suffered or is suffering from ischemia, the glycosylated protein of the extracellular matrix, preferably of the mammalian extracellular matrix, namely nidogen-1, is used for differentiating stem cells and/or progenitor cells, especially selected from the group consisting of stem cells, preferably embryonic stem cells, cardiac stem cells, cardiac progenitor cells, multipotent stem cells and combinations thereof, into differentiated, preferably fully differentiated, cardiomyocytes.

In a preferred embodiment, the differentiable, preferably undifferentiated cells, preferably selected from the group consisting of stem cells, preferably embryonic stem cells, cardiac stem cells, cardiac progenitor cells, multipotent stem cells and combinations thereof, preferably cardiac stem cells and/or cardiac progenitor cells are administrated to the human or animal subject in need thereof in form of agglomerates, preferably in a suspension or in a two or three dimensional scaffold. These agglomerates are called "embryoid bodies" in case of embryonic stem cells.

In a preferred embodiment of the present invention, the glycosylated protein nidogen-1 is administrated to the human or animal subject need thereof during or after suffering from ischemia. The term "after suffering" means preferably that the glycosylated protein, namely nidogen-1, is administered 1 hour to 15 years, preferably 1 hour to 6 months, preferably 1 minute to 6 weeks, preferably 1 minute to 1 day, preferably 1 minute to 6 hours, preferably 1 minute to 10 minutes, preferably 1 minute to 5 minutes, preferably 1 minute to 2 hours, preferably 1 minute to 4 hours, preferably 1 minute to 3 hours, preferably 5 minutes to 4 hours, preferably 10 minutes to 4 hours, preferably 15 minutes to 4 hours, preferably 2 to 6 weeks after the beginning of the ischemia of the heart in a human or an animal subject.

Preferably, nidogen-1 is administrated in a concentration of 20 to 100 µg/mL of administrated volume, preferably 30 to 70 µg/mL of administrated volume, preferably 50 µg/mL of administrated volume.

Preferably the amount of nidogen-1 administrated to a human subject within one administration is 1 µg to 1 mg, preferably 2 µg to 200 µg, preferably 2 µg to 100 µg, preferably 20 µg to 50 µg nidogen-1.

In a preferred embodiment of the present invention, the differentiable, preferably undifferentiated cells, preferably selected from the group consisting of stem cells, preferably embryonic stem cells, cardiac stem cells, cardiac progenitor cells, multipotent stem cells and combinations thereof, are administrated while being embedded in a two or three dimensional scaffold comprising the glycosylated protein nidogen-1.

In a preferred embodiment of the present invention, the glycosylated protein and/or the pharmaceutical composition is administrated into the subject in need thereof by injection directly into the peripheral circulation, heart muscle, left ventricle, right ventricle, coronary artery, cerebro-spinal fluid, neural tissue, ischemic tissue or post-ischemic tissue, in particular into the left ventricle, right ventricle, coronary artery, heart muscle tissue, ischemic tissue or post-ischemic tissue.

Further preferred embodiments are the subject-matter of the subclaims.

The present invention is illustrated by the figures and examples, below.

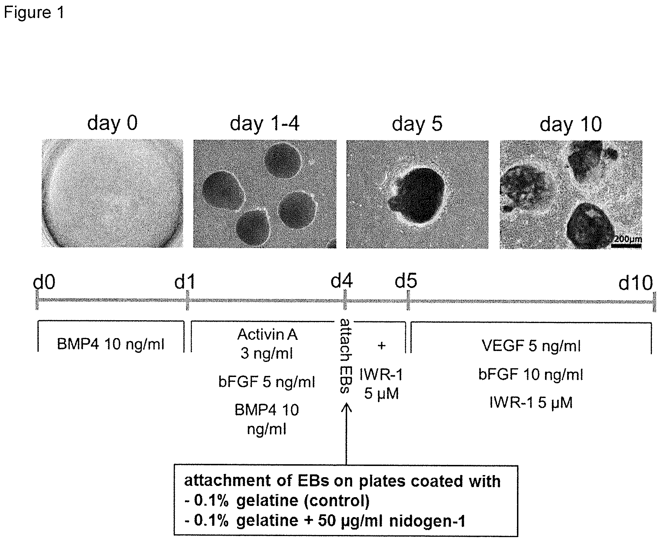

Figure 1 shows a modified cardiac differentiation protocol for spin embryoid bodies from human embryonic stem cells line H9 (the abbreviations used are explained in Examples 1 and 2, below).

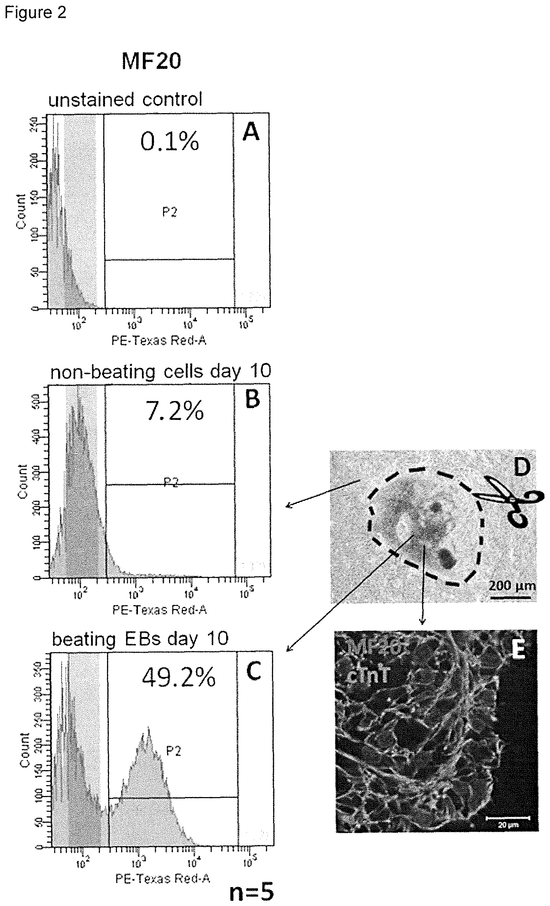

Figure 2 shows intracellular flow-cytometric analysis for sacromeric myosin (MF20 antibody), n = 5; Figure 2A: unstained control; Figure 2B: non-beating outgrown cells on day 10; Figure 2C: beating embryoid bodies on day 10; Figure 2D: samples for (B), (C), and (E) were dissected from the plate; Figure 2E: immunofluorescence image of the beating embryoid body on day 10 stained with sacromeric myosin (MF20) and cardiac troponin T. PE-Texas Red-A is a conjugate of Texas Red, also called sulforhodamine 101 acid chloride, with R-phycoerythrin. The abbreviation "EBs" used in Figure 2 means embryoid bodies.

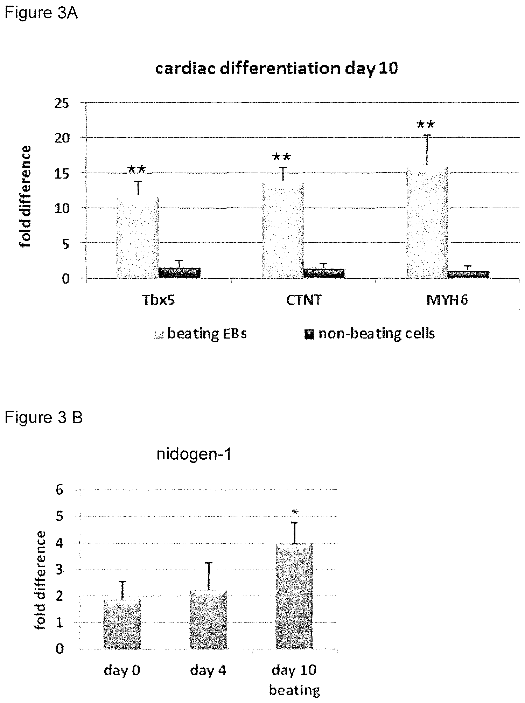

Figure 3 shows the result of the real time PCR-analysis; (A): of beating embryoid bodies (EBs) and non-beating cells at day 10 of cardiac differentiation with focus on the transcription factor Tbx5, cardiac troponin T and cardiac muscle myosin MYH6; (B): gene expression of nidogen-1 during cardiac differentiation.

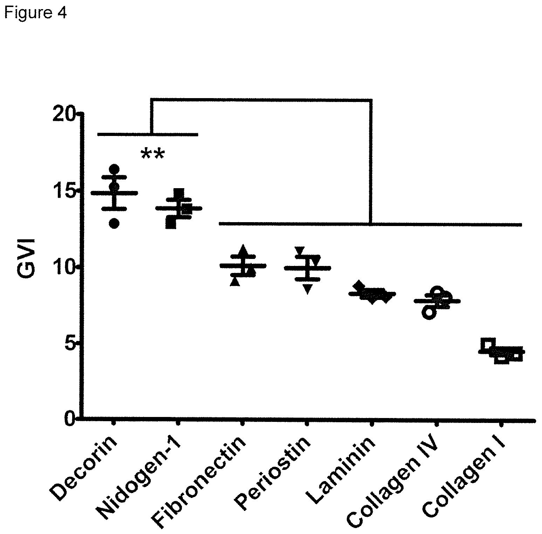

Figure 4 shows the comparison of the grey value intensities (GVI) of immunofluorescence images of beating embryoid bodies at day 10 of cardiac differentiation with focus on the extracellular matrix proteins decorin, nidogen-1, fibronectin, periostin, collagen I, collagen IV and laminin.

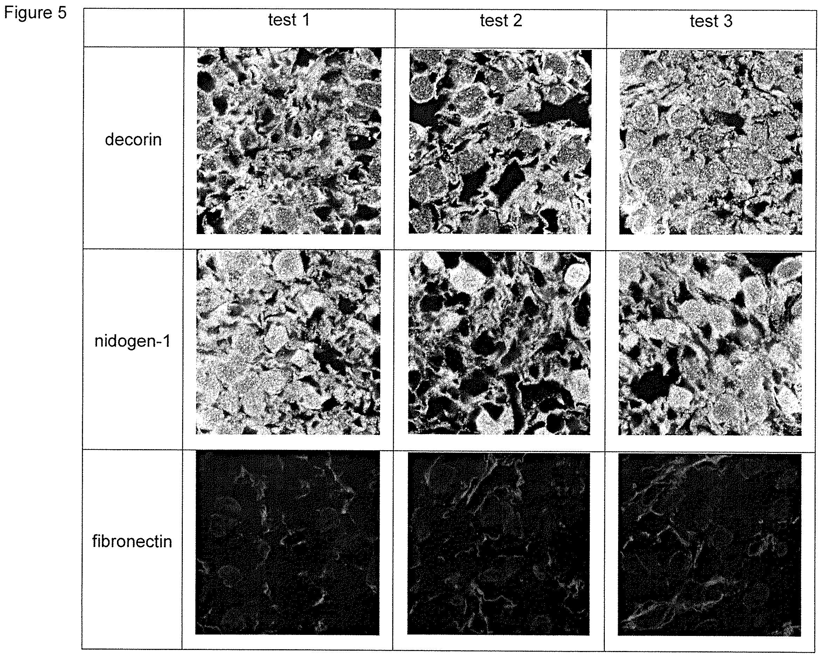

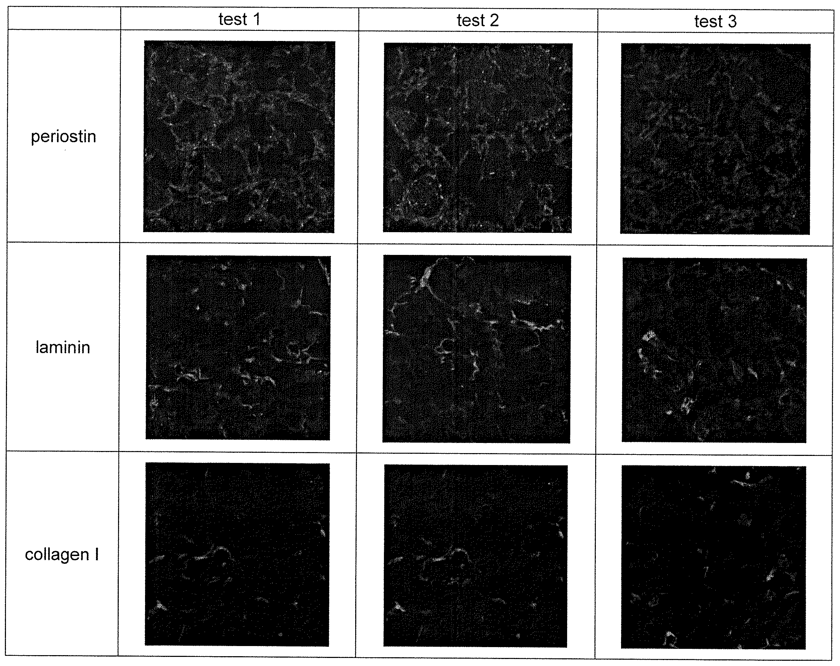

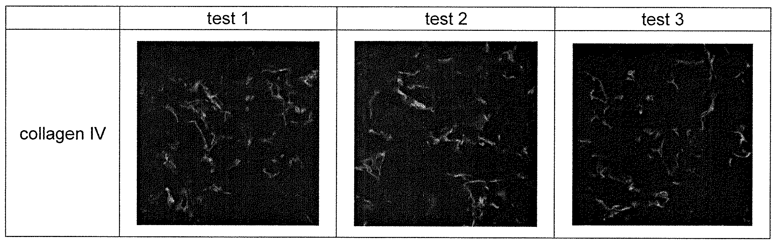

Figure 5 shows immunofluorescence images of beating embryoid bodies at day 10 of cardiac differentiation. The fluorescently stained (grey in figure 5) extracellular matrix proteins decorin, nidogen-1, fibronectin, periostin, laminin, collagen I and collagen IV were imaged with one of the channels of a immunofluorescence microscope. The differentiated cardiomyocytes with their sacromeric myosin also present in the sample have not been made visible. The fluorescently stained (grey in figure 5) extracellular matrix proteins have been found close to the differentiated cardiomyocytes.

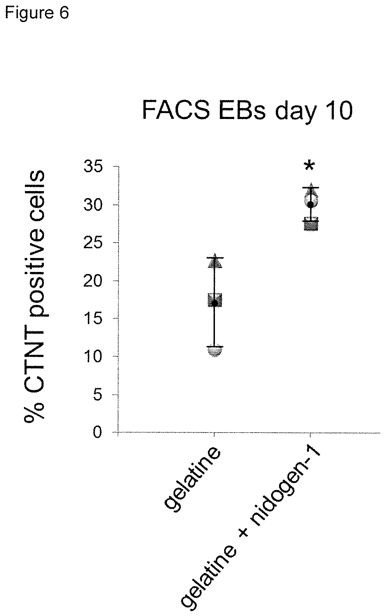

Figure 6 shows a significant increase in the percentage of CTNT-positive cells at day 10 of cardiovascular differentiation on a coating with 0.1 % by weight gelatine + 50 µg/ml nidogen-1 in comparison with the control coating (just 0.1 % by weight gelatine), n = 3, p < 0.05.

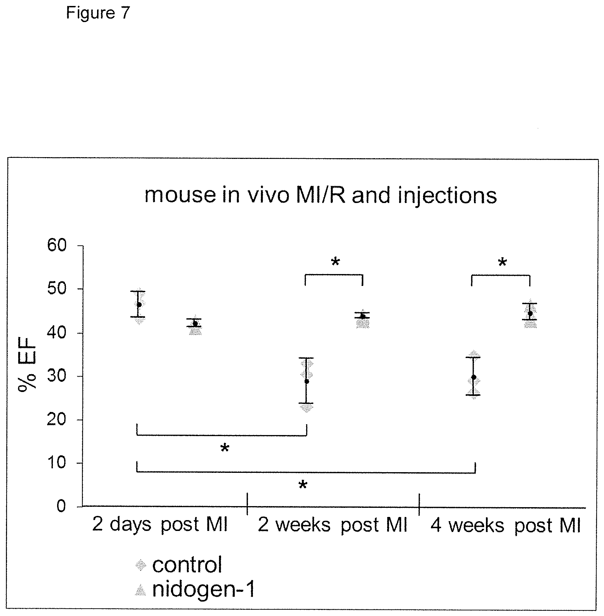

Figure 7 shows the percentage of ejection fraction (EF) of mice suffered a myocardial infarct (MI) and treated with nidogen-1 directly after suffering said myocardial infarct ("nidogen-1") and mice suffered a myocardial infarct and not treated with nidogen-1 ("control"). The percentage of the ejection fraction is significantly reduced in the control group (not injected with nidogen-1) two weeks and four weeks after the myocardial infarct compared to the time point two days after the myocardial infarct. In the nidogen-1 treatment group (administration of nidogen-1) this is not the case. The difference in ejection fraction between the nidogen-1 treatment group and the control group is significant at the time point 2 weeks after the myocardial infarct. The same is also true for the time point 4 weeks after the myocardial infarct.

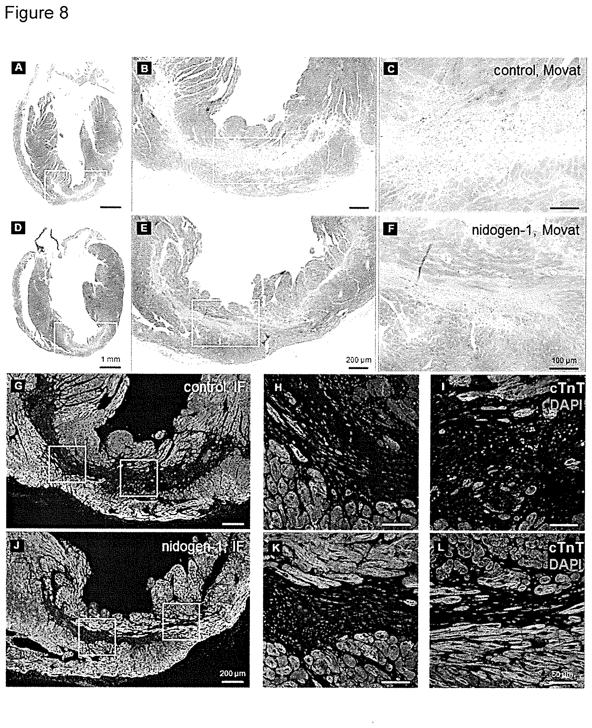

Figure 8 shows bright field images (Russel-Movat Pentachrome staining) and immunofluorescence (IF) images of paraffin-embedded tissue sections of mouse hearts that received nidogen-1 injections or control injections after a myocardial infarction and reperfusion (MI/R) event. White rectangles indicate the section that is shown in more detail in the image to the right. Figures 8A, 8B and 8C show Russel-Movat Pentachrome staining of a mouse heart that received control injections. Figures 8D, 8E and 8F show Russel-Movat Pentachrome staining of a mouse heart that received nidogen-1 injections. Figures 8G, 8H and 8I show immunofluorescence staining of cTnT (in light grey) and cell nuclei (DAPI, in darker grey) of a mouse heart that received control injections. Figures 8J, 8K and 8L show immunofluorescence staining of cTnT (in light grey) and cell nuclei (DAPI, in darker grey) of a mouse heart that received nidogen-1 injections.

### Examples:

### 1. Generation of embryoid bodies

hESCs (H9) (Thomson, J.A., et al., Embryonic stem cell lines derived from human blastocysts. Science, 1998.282(5391): p. 1145 to 1147) were cultured according to current protocols (WiCell Research Institute, Inc.) on MEF (mouse embryonic fibroblast) cells (Millopore (#PMEF-CFL)) (see also figure 1). For single cell suspension, the H9 cell colonies were washed with PBS (phosphate buffered saline), incubated with TrypLE Select (Invitrogen, life technologies) 5 min at 37°C, resuspended with a 1 ml pipette and passed through a cell strainer (40 µm, BD Falcon). The cell suspension was mixed with PBS in the ratio 1:1 and centrifuged (5 min, 1000 rpm). The cell pellet was resuspended in mTeSR1 culture medium (STEMCELL Technologies, Inc., Vancouver, Canada) with 10 ng/ml BMP4 (bone morphogenetic protein 4) and 10 ng/ml Rho-associated kinase (ROCK) inhibitor Y-27632 (Sigma, St. Louis, MO) to make a 600000 cells/ml suspension. 30000 cells (50 µl of the cell suspension) were dispensed per well in a 96-well plate with conical wells (96-Well Microplates, non-treated, conical bottom, Fisher Scientific). The cell suspension in the 96-well plate was centrifuged at 1000 rpm for 5 min and the plate was incubated overnight in a cell culture incubator.

### 2. Modified cardiac differentiation protocol

For cardiac differentiation, we used a modification of the differentiation protocols described in Yang (Human cardiovascular progenitor cells develop from a KDR+ embryonic-stem-cell-derived population. Nature, 2008. 453(7194): p. 524 to 528) and Willems et al. (Small-molecule inhibitors of the Wnt pathway potently promote cardiomyocytes from human embryonic stem cell-derived mesoderm. Circ Res, 2011. 109(4): p. 360 to 364). On day 1 of differentiation, after the overnight incubation, EBs (embryoid bodies) formed due to cell aggregation and were carefully transferred to ultra-low attachment 6-well plates (Corning incorporated, New York, Product #3471) with 1 ml pipettes. One 6-well can hold up to 200 EBs in 3 ml of StemPro 34 (Life technologies, Carlsbad, California, U.S.) + 3 ng/ml Activin A + 5 ng/ml bFGF (basic fibroblast growth factor) + 10 ng/ml BMP4 (stage 1 media). A change of the same media is required on day 2 or 3 of differentiation. The floating EBs were transferred to a 0.1 % Gelatin coated 6-well on day 4 with 2 ml of stage 1 media + 5 µM IWR-1 (4-(1,3,3a,4,7,7a-Hexahydro-1,3-dioxo-4,7-methano-2H-isoindol-2-yl)-N-8-quinolinyl-benzamide; as replacement of 150 ng/ml DKK-1). The EBs were incubated without moving the plate until the next day. On day 5, most of the EBs had attached to the dish and cells outgrew them. From day 5 to day 10 the attached EBs were differentiated in stage 2 media that consisted of StemPro 34 + 5 ng/ml VEGF (vascular endothelial growth factor) + 10 ng/ml bFGF + 5 µM IWR-1. A change of the same media was required on day 8 of differentiation. The attached EBs started beating on day 7 or 8 and were harvested for further analysis on day 10.

### 3. Analysis

To identify the differentiation status, flow-cytometric analysis were performed for sacromeric myosin (MF20 antibody) and immunofluorescence staining for cardiac troponin T (cTnT) and sacromeric myosin. Additionally, real-time PCR was utilized to monitor the expression of the transcription factor TbX5, cTnT and the alpha heavy chain of the cardiac muscle myosin (MYH6). The expression of the cardiac specific extracellular matrix during the differentiation process was analysed by real-time PCR and immunofluorescence.

### 4. Results

By employing the modified cell culture protocol, a high number of cells differentiated towards the cardiac lineage as identified by flow cytometric analysis (figure 2) and real-time PCR analysis (figure 3a) as well as immunofluorescence staining (figure 2e). It has been observed a robust and reproducible generation of spontaneously beating embryoid bodies (85% +/- 17.6 %). In addition, real-time PCR revealed a stage-specific and culture condition specific expression of extracellular matrix proteins with a significant increase in decorin and nidogen-1 expression during cardiac differentiation (figures 3b and 3c). Semi-quantitative analysis of immunofluorescence images (see figure 5) of beating embryoid bodies at day 10 of differentiation revealed are significantly higher amount of decorin and nidogen-1 as well as fibronectin compared to collagen IV and laminin in the beating embryoid bodies (figure 4 and table 1).

Table 1 shows the normalized grey value intensities of the images of figure 5.

**Table 1**

| protein | decorin | nidogen-1 | fibronectin | periostin | laminin | collagen IV | collagen I |
|---|---|---|---|---|---|---|---|
| test 1 | 16,405 | 14,831 | 9,136 | 11,042 | 8,017 | 8,048 | 4,398 |
| test 2 | 12,874 | 12,881 | 9,982 | 10,334 | 8,095 | 8,383 | 4,206 |
| test 3 | 15,240 | 13,846 | 11,219 | 8,573 | 8,817 | 7,097 | 4,970 |

### 5. In vitro-test

For the in vitro testing of the cardioinductive effect of nidogen-1, cell culture dishes were coated with 0.1 % by weight gelatine (as a control) or with 0.1 % by weight gelatine + 50 µg/ml human nidogen-1. Human nidogen-1 was recombinantly produced in Chinese Hamster Ovary (CHO) cells. The cardiovascular differentiation was conducted as described in the protocol in figure 1, starting with the human embryonic stem cell line H9. On Day 4 of differentiation the EBs were transferred to the coated culture dishes. Analysis of the efficiency of cardiovascular differentiation was conducted with fluorescence-activated cell sorting (FACS) analysis for the cardiomyocyte marker CTNT (cardiac Troponin T). The results of this analysis are shown in figure 6.

Figure 6 shows a significant increase in the percentage of CTNT-positive cells at day 10 of cardiovascular differentiation on the coating with 0.1 % by weight gelatine + 50 µg/ml nidogen-1 in comparison with the control coating (just 0.1 % by weight gelatine), n (number of runs) = 3, p < 0.05.

### 6. In vivo-test

In further in vivo experiments a mouse myocardial infarct (MI) model was used. After the controlled infarction event (MI/R prcedure), each mouse was injected with 50 µl of a mixture of an inert hydrogel and phosphate buffered saline (PBS) with or without 50 µg/ml nidogen-1 into the border zone of the infarct, also called infarct border region. Echocardiography revealed that the mice that received the nidogen-1 treatment showed a significant increase in the ejection fraction (EF; an important parameter for the cardiac function) in comparison to the control mice (no nidogen-1 injection) 2 and 4 weeks after the treatment (see also Figure 7).

### 7. Results for Histological H&E and Russel-Movat Pentachrome staining and immunofluorescence staining of mouse hearts

Histological staining (Russel-Movat Pentachrome) as well as immunofluorescence (IF) staining of the cardiomyocyte marker protein cTnT was performed on comparable sections of mouse hearts 4 weeks after MI/R ("myocardial ischemia/infarction and reperfusion") and injections of 50 µg/ml nidogen-1 or control injections in the peri-infarct zone (Fig. 8). Qualitative differences due to nidogen-1 or control injections can be seen in all of the conducted staining methods.

Russel-Movat Pentachrome staining was conducted to visualize the impact of the infarct and the subsequent injections with nidogen-1 or control injections on the ECM of the cardiac tissue. This staining clearly distinguishes between the ECM-rich infarct zone (light grey colors) and the surrounding tissue with intact cardiac muscle (dark grey). The light grey colors in the Russel-Movat Pentachrome staining indicate proteoglycans and collagen-rich ECM deposition in the infarct zone. The Russel-Movat Pentachrome staining identifies a lower degree of pathological remodeling as shown by a smaller infarct zone in the hearts that received nidogen-1 injections (Fig. 8D, 8E, 8F) compared to the control hearts (Fig. 8A, 8B, 8C). Collagen deposition in the nidogen-1-treated hearts appears more evenly distributed, whereas spatial accumulation of collagen deposits can be found in the control hearts.

The IF staining of the cardiomyocyte marker protein cTnT (in light grey), shows a clear distinction of the infarct zone and the non-infarcted surrounding cardiac tissue with cTnT-positive cardiomyocytes. In accordance with the results of the H&E and Russel-Movat Pentachrome staining, the qualitative analysis of the cTnT IF staining identifies a smaller area that lacks the functional contractile cardiomyocytes in the nidogen-1-treated mouse hearts (Fig. 8J, 8K, 8L) compared to the control treatment (Fig. 8G, 8H, 8I).

Therefore, qualitative analysis of these histological staining methods revealed that the nidogen-1 treatment enabled a better preservation and protection of the cardiac morphology compared to the control hearts. Furthermore, the extent of pathological remodeling is lower in the hearts that received nidogen-1 injections compared to the control injections.

### 8. In vivo mouse MI/R and ECM injection

A mouse model for myocardial infarction at the UCLA was used to identify the effect of myocardial injections with nidogen-1 post MI. Animal care and surgeries were performed in compliance with the University of California, Los Angeles Animal Research Committee under the protocols 2011-042 and 2013-057. Eight week old, female C57BL/6J mice, strain DLAMb6, were anesthetized using 2.0% isoflurane (Butler Schein, UK), placed on a heated surgical board and given flunixin (Flunixin Meglumine, Schering-Plough Animal Health, USA) 2.5 mg/kg subcutaneously. Under a dissecting microscope a midline cervical incision was made to expose the trachea for intubation with a PE-90 plastic catheter (Stoelting Company, USA). The catheter was connected to a Harvard minivent (Harvard Apparatus, Germany) supplying oxygen with a tide volume of 225 - 250 µl and a respiratory rate of 130 strokes/min. Surgical plane anesthesia was subsequently maintained with 1 - 1.5% isoflurane. A lateral incision was made in the fourth intercostal space. The heart was exposed and the left coronary artery was ligated intramurally 2 mm from its origin with a 9-0 nylon suture (Ethicon, USA). The suture was tied around a small piece of plastic tubing (PE-10) to occlude the coronary artery while allowing an easier and safer relief of the occlusion. This occlusion occurred for 45 minutes. During this time, a moist gauze pad was placed over the incision to maintain a sterile atmosphere. Ischemia was verified by the regional paleness of the myocardium that was no longer supplied with blood by the left coronary artery. Reperfusion was allowed by cutting the knot on the PE-10 tube and could be verified by the appearance of hyperaemia in the previously pale region. Due to reperfusion the infarcted region changed into a normal pinkish red color. This MI/R procedure was conducted on 10 mice.

After confirming that there was no bleeding, the animals received injections in the myocardial infarction border zone. The hydrogel and ECM injections were prepared as described in section 9, below. Five animals in the control group were injected with a total of 50 µl of HyStem® Hydrogel (Glycosan BioSystems, USA) + PBS per animal. In the nidogen-1 treatment group five animals received injections of HyStem® Hydrogel + PBS + 50 µg/ml nidogen-1 each of a total of 50 µl per animal. For each mouse the 50 µl total injection volume was divided into 10 µl injections at 5 sites in the LV myocardial infarction border zone.

The mice chests were closed in two layers. The ribs (inner layer) were closed with 6-0 coated vicryl sutures (Ethicon, USA) in an interrupted pattern. The skin was closed using 6-0 nylon or silk sutures (Covidien, UK) in a subcuticular manner. The anesthesia was stopped and the mice were allowed to recover for several minutes before the endotracheal tube was removed. For alleviation of pain the mice received intraperitoneal injections of Banamine (2.5 mg/kg) post-surgery.

### 9. Hydrogel and ECM preparation for post MI/R injections

All procedures for hydrogel + ECM injections were conducted under sterile conditions. The hydrogel consists of two components HyStem® and Extralink (Glycosan BioSystems, USA), which were prepared by dissolving the lyophilized solids in DG (degassed, deionized) water according to the manufacturer's instructions and by mixing HyStem and Extralink in a 1:4 volume ratio. Upon mixing of these two components gelation of the HyStem® Hydrogel occurred within 20 minutes. For each mouse a total of 50 µl of HyStem® Hydrogel + PBS ± ECM protein were needed. Therefore due to possible residues in the preparation tube or in the syringe 70 µl were prepared per mouse. For 70 µl of the injectable mix 8.4 µl consisted of the HyStem and Extralink mix. In the control group the remaining 61.6 µl were sterile PBS. In the nidogen-1 treatment group nidogen-1 was added for a total concentration of 50 µg/ml nidogen-1 accordingly.

### 10. Echocardiography and Vevo strain analysis

Conscious echocardiography was conducted on four different time points. A baseline echocardiography measurement was conducted on each mouse prior to the MI/R procedure. In serial nature further echocardiography measurements were conducted two days as well as two weeks and four weeks after the MI/R and myocardial injection procedure to track cardiac function, especially the ejection fraction as shown in Figure 7.

For echocardiographic imaging the high-frequency ultrasound imaging system Vevo 2100 (VisualSonics, Canada) was applied for non-invasively microimaging conscious mice in vivo with the MS400 transducer 18-38 MHz. Echocardiography was performed on conscious mice, as anesthesia can influence the heartbeat and thereby the experimental results. Myocardial performance was assessed via the Vevo LAB software, which enables regional and global wall motion tracing and offers the quantification of the strain.

Prior to the conscious echocardiography hair was removed from the mice's chest using a depilatory cream 3 - 5 min (Nair, Church & Dwight, USA). The animals were restrained on a platform and the echocardiogram was performed rapidly to minimize distress. Also the animals were released from the restraints if they showed signs of pain. The performed protocol was approved with the Chancellor's Animal Research Committee (ARC-OARO). The mice were sacrificed after the 4 week echocardiography and their hearts were explanted for histological investigation.

## Claims

1. A glycosylated protein of an extracellular matrix for use in a method of treating an ischemic heart of a human or animal subject in need thereof, wherein the glycosylated protein of the extracellular matrix is nidogen-1.

2. A pharmaceutical composition for use in a method of treating an ischemic heart of a human or animal subject in need thereof, comprising as a cardioinductive and/or cardioprotective agent a glycosylated protein of an extracellular matrix, which is nidogen-1.

3. The pharmaceutical composition for the use according to claim 2, comprising further at least one pharmaceutically acceptable carrier.

4. The glycosylated protein or pharmaceutical composition for the use according to any one of the preceding claims, wherein the treating of the ischemic heart comprises regenerating and/or protecting the heart tissue.

5. The pharmaceutical composition for the use according to any one of claims 2 to 4, wherein the glycosylated protein of the extracellular matrix is comprised in a hydrogel.

6. The pharmaceutical composition for the use according to any one of claims 2 to 5, wherein the glycosylated protein of the extracellular matrix is comprised in an electro-spun three-dimensional scaffold.

7. The pharmaceutical composition for the use according to any one of claims 2 to 6, wherein the glycosylated protein of the extracellular matrix is present together with differentiable cells selected from the group consisting of stem cells, preferably embryonic stem cells, cardiac stem cells, cardiac progenitor cells and combinations thereof.

8. The glycosylated protein or the pharmaceutical composition for the use according to any one of claims 1 to 7, wherein the glycosylated protein of the extracellular matrix is provided in injectable form for injection in the ischemic heart of the subject in need thereof.

9. The glycosylated protein or the pharmaceutical composition for the use according to any one of claims 1 to 8, wherein the glycosylated protein of the extracellular matrix is a recombinantly produced protein.

10. The glycosylated protein or pharmaceutical composition for the use according to any one of the preceding claims, wherein nidogen-1 is used as a cardioinductive agent and/or cardioprotective agent.

11. Method for the in vitro production of differentiated cardiomyocytes, wherein differentiable cells selected from the group consisting of stem cells, preferably embryonic stem cells, cardiac stem cells, cardiac progenitor cells and combinations thereof are cultivated with at least one biomolecule being or encoding a glycosylated protein of an extracellular matrix, which is nidogen-1, as to obtain differentiated cardiomyocytes, wherein nidogen-1 is used to differentiate the differentiable cells to differentiated cardiomyocytes.

12. Method according to claim 11, wherein the differentiable cells are cultivated on a plate coated with nidogen-1.

13. Use of nidogen-1 as cardioinductive agent able to differentiate differentiable cells selected from the group consisting of stem cells, preferably embryonic stem cells, cardiac stem cells, cardiac progenitor cells and combinations thereof to differentiated cardiomyocytes in the in-vitro production of differentiated cardiomyocytes.

14. The use according to claim 13, wherein the differentiable cells are cultivated on a plate cultured with nidogen-1.

## Patentansprüche

1. Glykolysiertes Protein einer extrazellulären Matrix zur Verwendung in einem Verfahren zur Behandlung des ischämischen Herzens von einem Menschen oder einem Tier, die dies benötigen, wobei das glykolysierte Protein der extrazellulären Matrix Nidogen-1 ist.

2. Arzneimittelzusammensetzung zur Verwendung in einem Verfahren zur Behandlung des ischämischen Herzens von einem Menschen oder einem Tier, die dies benötigen, umfassend ein glykolysiertes Protein einer extrazellulären Matrix als kardioinduktiver und/oder kardioprotektiver Wirkstoff, welcher Nidogen-1 ist.

3. Arzneimittelzusammensetzung zur Verwendung nach Anspruch 2, umfassend außerdem mindestens einen pharmazeutisch akzeptablen Träger.

4. Glykolysiertes Protein oder Arzneimittelzusammensetzung zur Verwendung nach einem der vorhergehenden Ansprüche, wobei die Behandlung des ischämischen Herzens eine Regeneration und/oder den Schutz des Herzgewebes umfasst.

5. Arzneimittelzusammensetzung zur Verwendung nach einem der Ansprüche 2 bis 4, wobei das glykolysierte Protein der extrazellulären Matrix in einem Hydrogel vorliegt.

6. Arzneimittelzusammensetzung zur Verwendung nach einem der Ansprüche 2 bis 5, wobei das glykolysierte Protein der extrazellulären Matrix in einem elektrogesponnenen dreidimensionalen Gerüst vorliegt.

7. Arzneimittelzusammensetzung zur Verwendung nach einem der Ansprüche 2 bis 6, wobei das glykolysierte Protein der extrazellulären Matrix gemeinsam mit differenzierbaren Zellen ausgewählt aus der Gruppe bestehend aus Stammzellen, vorzugsweise embryonalen Stammzellen, kardialen Stammzellen, kardialen Vorläuferzellen und Kombinationen davon vorliegt.

8. Glykolysiertes Protein oder Arzneimittelzusammensetzung zur Verwendung nach einem der Ansprüche 1 bis 7, wobei das glykolysierte Protein der extrazellulären Matrix in injizierbarer Form zur Injektion in das ischämische Herz des behandlungsbedürftigen menschlichen oder tierischen Patienten vorliegt.

9. Glykolysiertes Protein oder Arzneimittelzusammensetzung zur Verwendung nach einem der Ansprüche 1 bis 8, wobei das glykolysierte Protein der extrazellulären Matrix ein rekombinant hergestelltes Protein ist.

10. Glykolysiertes Protein oder Arzneimittelzusammensetzung zur Verwendung nach einem der vorhergehenden Ansprüche, wobei Nidogen-1 als kardioinduktiver Wirkstoff und /oder kardioprotektiver Wirkstoff verwendet wird.

11. Verfahren zur in-vitro Herstellung von differenzierten Kardiomyozyten, wobei differenzierbare Zellen, ausgewählt aus der Gruppe bestehend aus Stammzellen, vorzugsweise embryonalen Stammzellen, kardialen Stammzellen, kardialen Vorläuferzellen und Kombinationen davon, kultiviert werden mit mindestens einem Biomolekül, welches ein glykolysiertes Protein einer extrazellulären Matrix ist oder dieses kodiert, welches Nidogen-1 ist, um differenzierte Kardiomyozyten zu erhalten, wobei Nidogen-1 verwendet wird um die differenzierbaren Zellen zu differenzierten Kardiomyozyten zu differenzieren.

12. Verfahren nach Anspruch 11, wobei die differenzierbaren Zellen auf einer mit Nidogen-1 beschichteten Platte kultiviert werden.

13. Verwendung von Nidogen-1 als kardioinduktiver Wirkstoff, welches die differenzierbaren Zellen ausgewählt aus der Gruppe bestehend aus Stammzellen, vorzugsweise embryonalen Stammzellen, kardialen Stammzellen, kardialen Vorläuferzellen und Kombinationen davon zu differenzierten Kardiomyozyten in der in-vitro Herstellung von differenzierten Kardiomyozyten differenzieren kann.

14. Verwendung nach Anspruch 13, wobei die differenzierbaren Zellen auf einer Platte kultiviert mit Nidogen-1, kultiviert werden.

## Revendications

1. Protéine glycosylée d'une matrice extracellulaire à utiliser dans le cadre d'une méthode de traitement d'une ischémie cardiaque chez un sujet humain ou animal nécessitant ce type de traitement, où la protéine glycosylée de la matrice extracellulaire est le nidogen-1.

2. Composition pharmaceutique à utiliser dans le cadre d'une méthode de traitement d'une ischémie cardiaque chez un sujet humain ou animal nécessitant ce type de traitement, comprenant un agent cardioinducteur et/ou cardioprotecteur sous la forme d'une protéine glycosylée d'une matrice extracellulaire, qui est le nidogen-1.

3. Composition pharmaceutique à utiliser selon la revendication 2, comportant au moins un porteur de qualité pharmaceutique.

4. Protéine glycosylée ou composition pharmaceutique à utiliser selon les revendications précédentes, où le traitement de l'ischémie cardiaque comporte la régénération et/ou la protection des tissus cardiaques.

5. Composition pharmaceutique à utiliser selon les revendications 2 à 4, où la protéine glycosylée de la matrice extracellulaire est incluse dans un hydrogel.

6. Composition pharmaceutique à utiliser selon les revendications 2 à 5, où la protéine glycosylée de la matrice extracellulaire est incluse dans une structure tridimensionnelle électrofilature.

7. Composition pharmaceutique à utiliser selon les revendications 2 à 6, où la protéine glycosylée de la matrice extracellulaire est présente avec des cellules différenciables sélectionnées dans le groupe composé de cellules souches, de préférence des cellules souches embryonnaires, des cellules souches cardiaques , des cellules progénitrices cardiaques et des combinaisons de ces cellules.

8. Protéine glycosylée ou composition pharmaceutique à utiliser selon les revendications 1 à 7, où la protéine glycosylée de la matrice extracellulaire est fournie sous forme injectable pour injection dans l'ischémie cardiaque du sujet nécessitant ce type de traitement.

9. Protéine glycosylée ou composition pharmaceutique à utiliser selon les revendications 1 à 8, où la protéine glycosylée de la matrice extracellulaire est une protéine produite par recombinaison.

10. Protéine glycosylée ou composition pharmaceutique à utiliser selon les revendications précédentes, où le nidogen-1 est utilisé en tant qu'agent cardioinducteur et/ou cardioprotecteur.

11. Méthode de production in vitro de cellules myocardiques différenciées, où les cellules différenciables sélectionnées dans le groupe composé de cellules souches, de préférence des cellules souches embryonnaires, des cellules souches cardiaques , des cellules progénitrices cardiaques et des combinaisons de ces cellules sont cultivées avec au moins une biomolécule constituant ou codant une protéine glycosylée d'une matrice extracellulaire, le nidogen-1, pour obtenir des cellules myocardiques différenciées, où le nidogen-1 est utilisé pour différencier les cellules différenciables des cellules myocardiques différenciables.

12. Méthode selon la revendication 11, où les cellules différenciables sont cultivées sur une plaque recouverte de nidogen-1.

13. Utilisation de nidogen-1 en tant qu'agent cardioinducteur capable de différencier les cellules différenciables sélectionnées dans le groupe composé de cellules souches, de préférence des cellules souches embryonnaires, des cellules souches cardiaques , des cellules progénitrices cardiaques et des combinaisons de ces cellules, des cellules myocardiques différenciées dans la production in vitro de cellules myocardiques différenciées.

14. Utilisation selon la revendication 13, où les cellules différenciables sont cultivées sur une plaque cultivant du nidogen-1.
